# EUROPEAN PATENT APPLICATION

(11) **EP 4 483 829 A1**
(43) Date of publication of application: **01.01.2025**
(21) Application number: 23205994.9
(22) Date of filing: 26.10.2023
(51) Int. Cl.: A61B 18/18, A61B 18/20, A61B 18/00

(54) **LIGHT-BASED HAIR OR SKIN TREATMENT**

(30) Priority: 28.06.2023 WO PCT/CN2023/103106
(71) Applicant: Koninklijke Philips N.V., 5656 AG Eindhoven (NL)
(72) Inventor: KELLY, Declan Patrick, 5656 AG Eindhoven (NL); GU, Wei, 5656 AG Eindhoven (NL); MENG, Jiaxu, 5656 AG Eindhoven (NL); PALERO, Jonathan Alambra, 5656 AG Eindhoven (NL); NUIJS, Antonius Maarten, 5656 AG Eindhoven (NL)
(74) Representative: Philips Intellectual Property & Standards

(57) **Abstract**

A device is provided for hair removal or treatment of skin in which treatment light is delivered through an output window, and at least a portion of the output window has an electrically controllable optical transmittance for the treatment light so that the effective size of the treatment head can be adjusted to be suitable for different treatment regions.

## Description

### FIELD OF THE INVENTION

This invention relates to a device for light-based hair removal or treatment of skin.

### BACKGROUND OF THE INVENTION

For hair removal, light based epilators are well-known. People use hair epilators or depilators to remove unwanted hair. Typical target areas for women are the face, armpit, arm, leg, bikini line, and body. Men use light-based epilators also on the chest and back.

These different target areas have different surface contours, and thus typically need different treatment heads. For example, the Philips Lumea epilator has four different attachments, termed Body, Facial, Armpit and Bikini.

Managing multiple treatment heads is a burden to customers. It would be more convenient to have a single configurable treatment head that can be used for all target locations.

One solution is disclosed in WO 2020/035405, which discloses a LED-based photo-epilator with a reconfigurable treatment head. The treatment head has three surfaces with LEDs on them. A selected one of surfaces is turned on for hair removal. This solution is only suitable for LED epilators. However, mainstream hair epilators are still based on the filtered output of a flashlamp, known as Intense Pulsed Light or IPL.

IPL devices also have the advantage that they potentially enable a skin rejuvenation mode, to be combined with a hair removal mode or as mode in a stand-alone device.

Thus, there is still no solution for a low cost and easily reconfigurable epilator, or indeed more generally a reconfigurable skin treatment device. It would be particularly interesting to provide a solution suitable for implementation in a light based epilator or skin treatment device.

### SUMMARY OF THE INVENTION

The invention is defined by the claims.

According to examples in accordance with an aspect of the invention, there is provided a device for hair removal or treatment of skin, comprising:
a light source for generating treatment light;
a controller for controlling the light source; and
an output window through which the treatment light is delivered to a user's skin, in use,
wherein at least a portion of the output window has an electrically controllable optical transmittance for the treatment light between a first state and second state, wherein the portion of the output window is more optically transmissive in the first state than in the second state.

This controllable output window enables the device to be configured for different skin regions, without needing to change attachments or provide mechanically moving internal parts. A portion of the output window can be change from a first state e.g., transparent to a second state e.g., substantially non-transparent (reflective or opaque). Preferably the transparent area of the output window can be controlled to multiple sizes.

The second state (hereinafter also referred as non-transmissive state) may be fully non-transmissive or it may simply be less transmissive than the first state (hereinafter also referred as transmissive state) so that a reduced light output results in a selected portion of the output window. Preferably, the second state allows transmission of at most 10% of the fluence (at the skin surface) compared to the first state.

In one set of examples, the non-transmissive state is a light blocking state. In this case, the portion of the output window may be implemented as a switchable glass.

In the case of switchable glass, the front of the treatment head (facing the user's skin) is switchable glass that can be controlled electrically to make parts of it opaque. In this way, the active area of the treatment head can be adapted under electrical control.

In another set of examples, the non-transmissive state is a light reflecting state. In this case, the portion of the output window may be implemented as a switchable mirror. In the case of switchable mirrors, the front of the treatment head is a switchable mirror with the mirror surface facing towards the inside of the device. The switchable mirror can be electrically controlled so that parts of the window reflect light back into the device, where it is reflected again by an optical chamber of the light source and out through the output window. Some controllable mirrors can also tune the light spectrum.

The device for example comprises an optical chamber with a curved rear reflector, wherein the switchable mirror is configured to reflect the treatment light to the curved rear reflector. The curved rear reflector ensures the reflected light follows a path (with one or more reflections first) to the output window.

The output window for example comprises an inner transparent support layer and an outer switchable layer with said electrically controllable transmittance. This protects the switchable layer from the heat generated internally.

The output window for example comprises a pixelated device with per-pixel control of optical transmittance. This enables fine control of the shape and size of the output window that is rendered optically transmissive.

The controller is for example configured to adjust the light source power in dependence on an area of the output window that is optically transmissive. When the window size is decreased, the power can be proportionally decreased (or with a slightly higher power to compensate the reflection loss) to give the same output fluence (in J/cm²). The controller is for example configured to maintain a constant output fluence for a given treatment area when adjusting the optical transmittance.

The controller is for example configured to adjust the light source power in dependence on the skin region to be treated. Different lighting power is suitable for different skin regions.

The device may further comprise a detection system to detect which skin regions have already been treated, and the controller is configured to control the optical transmittance in dependence on which skin regions, within a current treatment area covered by the device, have already been treated.

When (known) sensors are used to detect the device location on the skin and track regions that have already been treated, when the device moves to a partially treated region, the output window can be reduced to only cover the missed part.

The device can be used for the purpose of light-based hair removal or skin treatment. A suitable light source is for example a laser, flashlamp or an array of LEDs. In the case of a flashlamp, the light must pass through an optical filter; the filtered output is known as IPL or Intense Pulsed Light.

The invention also provides a method of controlling a device for hair removal or treatment of skin, the device comprising a treatment light source for generating treatment light and an output window through which the treatment light is delivered to a user's skin, in use, the method comprising:
electrically controlling an optical transmittance of at least a portion of the output window for the treatment light between a first state and a second state, wherein the portion of the output window is more optically transmissive in the first state than in the second state.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a device for hair removal or treatment of skin;
Fig. 2 shows a typical IPL treatment head; and
Fig. 3 shows three possible ways to change the area of the output window.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device for hair removal or treatment of skin in which treatment light is delivered through an output window, and at least a portion of the output window has an electrically controllable transmittance for the treatment light so that the effective size of the treatment head can be adjusted to be suitable for different treatment regions.

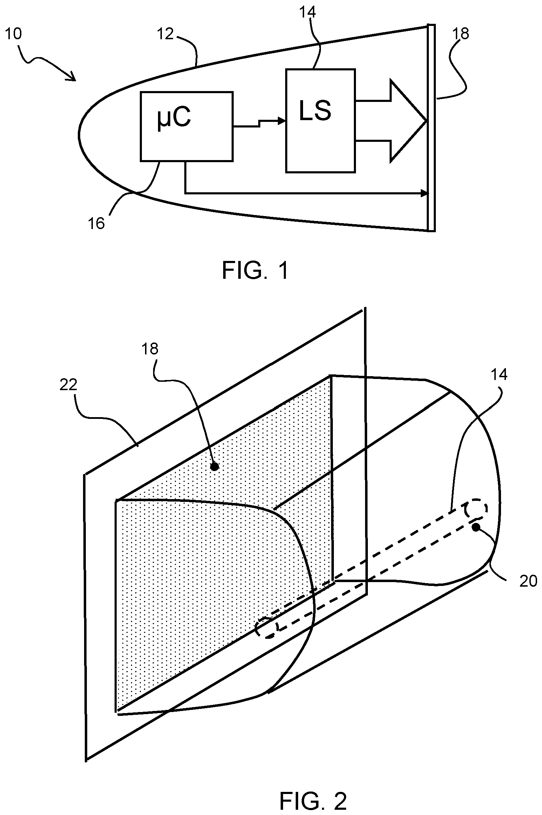

Fig. 1 shows a device 10 for hair removal or treatment of skin, and in particular shows an IPL device as example. The device comprises an outer housing 12 which contains a flashlamp 14 for generating treatment light in this example. A controller 16 is provided for controlling the light source. The light source delivers treatment light to the skin of the user (and hence to hairs of the user on the skin surface) through an output window 18.

In accordance with the invention, at least a portion of the output window 18 has an electrically controllable optical transmittance for the treatment light (i.e., for light of a frequency corresponding to the frequency of the treatment light). The optical transmittance is controllable between a first state and second state. The first state is more optically transmissive, e.g., transparent compared to the second state, e.g., light-blocking. The output window has a controllable area which is in the first state, i.e., optically transmissive, and this enables the device to be configured for different skin regions, without needing to change attachments or provide mechanically moving internal parts. The second state for example allows transmission of at most 10% of the fluence (at the skin surface) compared to the first state.

A typical IPL treatment head is illustrated in Fig. 2. The light source 14 for example comprises a Xenon flashlamp. The flashlamp is surrounded by reflecting surfaces, including a curved rear reflector surface 20 which reflects light to the output window 18. The curved reflector shapes the light from the flashlamp into a more or less parallel beam. All light from the Xenon flashlamp reaches the front plate 22 which includes the output window 18, either directly or after one or more reflections.

The invention provides a variable area of an optically transmissive part of the output window 18. With the output window fully open (i.e., fully optically transmissive), the device is for example in a mode for applying treatment to the largest target area, for example on the arm.

To treat a smaller region, for example in a mode for applying treatment to the face, a smaller optically transmissive output window is needed. This is achieved by electrically controlling the output window 18 to only allow light from a part of the total window area to exit the device.

A first option is to make part of the window opaque to block the light. In this case, the blocking part will absorb the light and heat up so the design needs to take into account this thermal heating.

An alternative approach is to electrically turn part of the output window into a mirror that reflects light back into the device where it will be reflected again (from the reflective surface 20 behind the flashlamp) back to the output window.

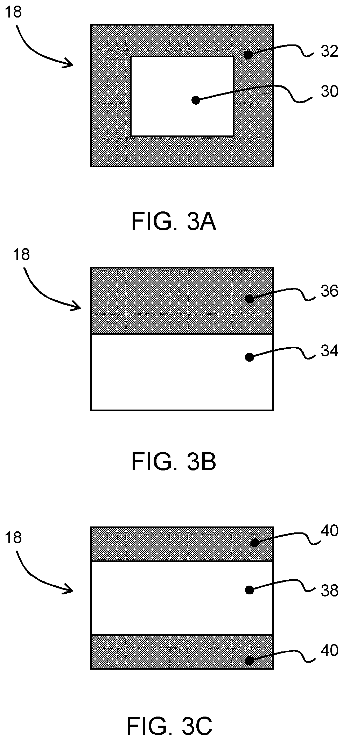

Fig. 3 shows three possible ways to change the area of the optically transmissive part of the output window. In the first example of Fig. 3A, there is a central window area 30 that is always optically transmissive, and a surrounding area 32 that controllable between transmissive and reflective or blocking states.

In the second example of Fig. 3B, there is a lower window area 34 that is always optically transmissive, and an upper area 36 that is controllable between transmissive and reflective or blocking states.

In the third example of Fig. 3C, there is a central band area 38 that is always optically transmissive, and upper and lower bands 40 that are controllable between transmissive and reflective or blocking states.

There may be only two states, such as a fully transmissive window and a window configured as shown in the three examples of Fig. 3. However, instead, the output window may be pixelated so that it can be driven to any desired pattern of transmissive and non-transmissive states, Thus, rather than having only two distinct states, there may be any number of states, such as four states (i.e., different sizes of transmissive area) corresponding to the sizes of the four known separate treatment heads known in the prior art, as mentioned above. A fully flexible control of the pixelated output window may even be provided to enable additional functionality as discussed below.

In one preferred example, there is a rectangular optically transmissive opening as shown in Fig. 3A, and the size of the rectangular opening of the output window is controllable.

When the optical properties can be controlled at a pixel level, the light treatment may also be controlled to provide light blocking of localized spots such as moles, freckles, and pigmented spots. Furthermore, it can enable adaptive treatment where the treatment light intensity can be adjusted based on a controllable window transparency or opaqueness.

For each configuration with a reduced area transmissive part of the output window, the system may be controlled to reduce the light source power such as to deliver a desired fluence through the output window (in J/cm²) based on the known properties of the system in terms of the amount of light that exits the output window. This can be determined as part of a calibration procedure during development of the device.

It is known to incorporate a sensor to track movement of a light-based hair removal or skin treatment device. For example, the device may have a mode in which the user moves the device over the skin while a sensor tracks the location and displacement.

The sensor is for example an optical sensor for tracking movements based on observed changes of a captured image. The working mechanism of such an optical sensor is similar to an optical mouse (a mouse embedded with an optical sensor) for a computer used tracking the movement of the mouse. Tracking of location could also be achieved using a camera embedded in the device.

Typically, a user will move the device over the treatment area multiple times to ensure full coverage. When the user moves the device over a region which includes a previously treated portion and a previously untreated portion, the system can adjust the size and shape of the optically transmissive part of the output window to avoid repeat treatment of the already treated portion. The light source power can again be adjusted to ensure the required fluence is delivered through the reduced area output window. In this way, over-treatment or under-treatment of different skin regions can be prevented.

The curved reflector shown in Fig. 2 prevents the possibility, when using a reflective output window, for light to be trapped between two parallel mirrors and reflected back and forth multiple times. Instead, after one or few reflections, the light will escape to the transmissive part of the output window.

The electrically controllable glass or mirror component typically has an operating temperature range, for example -10°C to 60°C for the "e-Transflector" electrically switchable transflective mirror of KentOptronics. The electrochromic layer is therefore applied preferably on the outside of the output window, with an additional transparent material (such as a base glass plate) to insulate it from the heat of the light source.

In current IPL devices, the outside of the output window will never reach 60°C since this is above the maximum external, skin-contacting, operating temperature to avoid user pain. If the additional base plate is not desired, the temperature in the cavity may instead be designed to remain below the operating temperature of the electrochromic material.

Different power levels as well as different output window areas may be appropriate for different skin regions.

By way of example:
an output of 5.5J/cm² over 4 cm² may be provided for body treatment;
an output of 6.5 J/cm² over 3 cm² may be provided for armpit or bikini treatment;
an output of 6 J/cm² over 2 cm² may be provided for the face. The cut-off wavelength may also be different.

Electrical to optical efficiency is important since the generation of heat is the main cause for a low flash rate. However, in the case of an output window with reduced transmissive area, and consequently reduced power, the overall heat generated per flash will be lower than the case of the full output window. For example, based on the numbers above 6 J/cm² over 2 cm² is equivalent to to 12 J whereas 5.5 J/cm² over 4 cm² is equivalent to 22 J. Thus, the smaller output windows will use lower overall power and therefore generate less heat.

The invention may be applied to IPL epilators and skin treatment devices. However, it may also be applied to other (optical) epilator designs for example using LEDs or lasers. It may also be applied to other skin treatment devices using light.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A device (10) for hair removal or treatment of skin, comprising:
a light source (14) for generating treatment light;
a controller (16) for controlling the light source; and
an output window (18) through which the treatment light is delivered to a user's skin, in use,
wherein at least a portion (30,36,40) of the output window (18) has an electrically controllable optical transmittance for the treatment light between a first state and a second state, wherein the portion of the output window is more optically transmissive in the first state than in the second state.

2. The device of claim 1, wherein the second state is a light blocking state.

3. The device of claim 2, wherein the portion of the output window comprises a switchable glass.

4. The device of claim 1, wherein the second state is a light reflecting state.

5. The device of claim 4, wherein the portion of the output window comprises a switchable mirror.

6. The device of claim 5, comprising an optical chamber with a curved rear reflector (20), wherein the switchable mirror is configured to reflect the treatment light to the curved rear reflector.

7. The device of any one of claims 1 to 6, wherein the output window comprises an inner transparent support layer and an outer switchable layer with said electrically controllable transmittance.

8. The device of any one of claims 1 to 7, wherein the output window comprises a pixelated device with per-pixel control of optical transmittance.

9. The device of any one of claims 1 to 8, wherein the controller (18) is configured to adjust the light source power in dependence on an area of the output window that is optically transmissive.

10. The device of claim 9, wherein the controller is configured to maintain a constant output fluence for a given treatment area when adjusting the optical transmittance.

11. The device of any one of claims 1 to 10, wherein the controller is configured to adjust the light source power in dependence on the skin region to be treated.

12. The device of any one of claims 1 to 11, further comprising a detection system to detect which skin regions have already been treated, wherein the controller is configured to control the optical transmittance in dependence on which skin regions, within a current treatment area covered by the device, have already been treated.

13. The device of any one of claims 1 to 12, wherein the light source comprises a flashlamp.

14. A method of controlling a device for hair removal or treatment of skin, the device comprising a treatment light source for generating treatment light and an output window through which the treatment light is delivered to a user's skin, in use, the method comprising:
electrically controlling an optical transmittance of at least a portion of the output window for the treatment light between a first state and a second state, wherein the portion of the output window is more optically transmissive in the first state than in the second state.
